# EUROPEAN PATENT SPECIFICATION

(11) **EP 3 618 788 B1**
(45) Date of publication and mention of the grant of the patent: **05.01.2022**
(21) Application number: 18716254.0
(22) Date of filing: 06.04.2018
(51) Int. Cl.: A61F 13/20, A61L 15/58

(54) **TAMPON AND METHOD FOR PRODUCING A TAMPON**
TAMPON UND VERFAHREN ZUR HERSTELLUNG EINES TAMPONS
TAMPON ET PROCÉDÉ DE FABRICATION D'UN TAMPON

(30) Priority: 04.05.2017 DE 102017004322
(43) Date of publication of application: 11.03.2020
(73) Proprietor: Rauscher Consumer Products GmbH, 2525 Schönau/Triesting (AT)
(72) Inventor: PÖTSCH, Ernst, 2525 Schönau/Triesting (AT); MILZ, Claudia, 2542 Mannerdorf/Leithagebirge (AT)
(74) Representative: Seranski, Klaus
(86) International application number: PCT/EP2018/058853
(87) International publication number: WO 2018/202382

(56) References cited:
- EP-A1- 2 417 953
- EP-A2- 0 685 213
- WO-A1-2014/015192

## Description

The invention relates to a method for producing a tampon as specified in the precharacterizing portion of claim 1.

For a long time tampons have been used to absorb body fluid in wound care, significantly however in connection with female menstruation. A simple tampon, consisting of a plug made of an absorbent material, a covering material in the form of gauze, a retrieval cord and a funnel made of permeable material is already described in Patent Specification 624395 issued by the Imperial Patent Office in 1936.

Corresponding tampons are still being developed to this day. For example, in order to highlight the individual development steps, reference is made to German Patent Specification 804835 from 1949, German Laid-Open Document 2722802 A1 from 1977, German Patent Application 3519515 A1 from 1985, the international patent application with publication number WO 2001/24729 A2 from 2001, the European patent application with publication number 2298259 A from 2010 and the subsequently published European Patent Application 2431014 A1.

Even in Patent Specification 624395 it is pointed out that covering of the absorbent body is required in order to prevent residues of the fibrous, absorbent material from remaining in the body cavity.

Methods for applying the covering material are described, for example, in DE 3519515. Numerous improvements with regard to the complete utilisation of the absorption capacity of the absorbent material of the tampon, the slide characteristics, the comfort of wearing, the leakage protection and the like have been proposed. For example, reference is made in this connection to EP 2298259. According to this specification the formation of a depression in a dome forming the insertion end of the tampon is proposed so as to thus improve the introduction of body fluid into the core of the absorbent body and to prevent premature saturation of the outer region of the absorbent body.

EP 2417953 A1 discloses a tampon having an absorbent body including on its outer surface an applied portion to which an agent is applied. The agent of the known tampon is a mixture of an active pharmaceutical ingredient, a first water soluble carrier that carries the active pharmaceutical ingredient and is a main ingredient of the agent, and a second water soluble carrier that carries the active pharmaceutical ingredient and has a melting point lower than that of the first water soluble carrier.

EP 2685213 A discloses a tampon comprising a spirally wound strip of absorbent core material with a thermally bondably non-woven cover web of essentially continuous fibres adhered to a surface of said absorbent core material to form a laminate with said fibrous non-woven web cover material forming an exterior surface of said tampon.

WO 2014/015192 A1 discloses a tampon comprising an absorbent pledget and a time lapse indicator associated therewith.

The object underlying the invention is to improve the properties of a tampon, in particular a female hygiene tampon.

According to the invention, this object is achieved by a further development of the known tampon as specified in the appended claims. Preferred embodiments of the invention are specified in the dependent claims.

In order to obtain the desired effects and functions according to this invention it is generally necessary for the portion of functional material in the overall weight of the tampon to be 0.5 % by weight or more, in particular 0.8 % by weight or more, preferably 1.0 % by weight or more. In order to avoid any undesirable effect upon the absorption properties and/or the desired properties of the covering material it is advantageous here if the portion of functional material in the overall weight of the tampon is 6 % by weight or less, preferably 4 % by weight or less, in particular 3 % by weight or less.

With regard to the desired functions or effects, the functional material may comprise polypropylene, polyethylene, polyethylene terephthalate (PET), starch, a foam, natural substances and/or dyes. According to the invention the functional material is provided with adhesive properties which improve the adhesion to the absorbent material and/or the covering material and/or support the adhesion between the absorbent material and the covering material. Furthermore, the adhesive properties may counter undesired distribution of the functional material over larger volume areas of the tampon.

In particular when visual effects are to be achieved, it has proven to be advantageous if the functional material can be distinguished from the absorbent material and/or the covering material in terms of colour. In other embodiments the functional material may also be made to be transparent in order to prevent the appearance of the tampon being affected by the functional material if other functional properties, such as for example fluid distribution, leakage protection, slide characteristics or the like are to be supported by the functional material.

If the properties of the functional material, in particular the colour, are dependent upon the temperature, the storage time, the humidity, the chemical environment or the like, the functional material can also perform a warning function by means of which risks during use, an exceeded storage time, excessive moisture, unfavourable chemical conditions and/or an excessive temperature effect are indicated.

In order to prevent undesirable distribution of the functional material over larger volume areas, or in accordance with the desired localisation of the functional material, the softening temperature of the latter is in the range of 80° C or more, preferably 100° C or more. The functional material can be processed without damaging the absorbent material and/or the covering material because the softening temperature of the functional material is 200° C or less, in particular 150° C or less. The softening point in accordance with this application is the softening point according to ASTM E28 in glycerine.

The processing temperature for the functional material is 80° C or more, in particular 110°C or more, but it should be less than 280° C, in particular less than 180°C.

The viscosity of the functional material is advantageously set such that it is 100 mPas or more, in particular 500 mPas or more at the processing temperature, but in order to ensure trouble-free application it should be no more than 1,000,000 mPas, in particular no more than 10,000 mPas, preferably 7.000 mPas or less.

The functional material can also have adjustable swelling behaviour. It may be, for example, a functional material which changes its shape due to the effect of other factors, such as for example moisture, temperature or the like, so as to thus improve the performance characteristics or to perform a warning function. The functional material may also comprise additives such as for example aloe, camomile, activated carbon or the like.

In terms of a desired distribution of the body fluid to be absorbed by the tampon and optimal utilisation of the absorptive capacity of the tampon, it has proven to be advantageous if strips of functional material made of a hydrophobic functional material are provided on the outer boundary surface of the covering material and/or between the covering material and the absorbent material in order to distribute fluid. The fluid striking the outer surface of the tampon is then conveyed away with the aid of the strips of functional material so that the absorptive capacity of the tampon can be completely utilised.

In addition or alternatively, provision can be made such that in the winding, which is generally pressed in the course of the production process, at least one shell made of hydrophobic functional material is formed with a diameter of 1 mm or more, preferably 1.5 mm or more, and/or 7 mm or less, in particular 5 mm or less, the shell extending from edges facing the insertion end of the tampon with a convex boundary surface towards the end of the tampon facing away from the insertion end, and has a depth of 0.5 mm or more, preferably 1 mm or more, but at most 7 mm, in particular at most 5 mm.

The fluid flowing within the absorbent body towards the end of the tampon facing away from the insertion end is collected in the corresponding shells so that the volume above the shell or on the side of the shell facing the insertion end can be better utilised to absorb body fluid. A tampon according to the invention can have two, three or more shells made of hydrophobic functional material arranged one behind the other in the axial direction and/or offset to one another in the radial direction.

Advantageous distribution of the body fluids in the absorbent body can also be achieved by different distribution of the functional material. It is conceivable, for example, to apply loop- or wave-shaped structures of the functional material in the region of the suction body or only in the region of the covering material. Furthermore, shell-like functional material sections can be provided only in the region of the covering material or of an individual winding of the absorbent body. In this case the shell has a curve pointing towards the end of the tampon facing away from the insertion end. In other embodiments the curve can also point towards the insertion end. In this connection the expression "insertion end" designates the end of the tampon which is generally provided with a dome.

Particularly reliable leakage protection is achieved if at least one preferably continuous layer of hydrophobic functional material is formed in the region of the end of the winding facing away from the insertion end, which layer particularly preferably extends approximately in a plane running perpendicularly to the winding axis or tampon axis.

In a method according to the invention for producing a tampon according to the invention, a covering material is applied to a material web made of absorbent material, the absorbent material that is thus provided is wound up relative to a winding axis to form a winding so that the covering material at least partially covers the outer boundary surface of the winding, and the winding presses in the axial and/or radial direction. Such methods are described, for example, in EP 2740448 A1. The disclosure content with regard to the individual features of corresponding methods and to the features of devices for implementing such methods is herewith incorporated into this description by explicit reference.

A method according to the invention is essentially characterised in that the absorbent material and/or the covering material is provided with the functional material before and/or during the winding process. When the absorbent material, which is generally in the form of a strip of cotton wool, is provided with the functional material, this material can be provided with the functional material when the strip of cotton wool passes out of a carding machine or during a laying process and/or when passing into the tampon machine. In addition or alternatively, it is also conceivable to separate the provision of the absorbent material with the functional material from the other process steps and to bring this about in a separate process step independently of the other process steps.

Needless to say, it is also conceivable to produce a material web already provided with the functional material, which has adhesive properties, from absorbent material, such as for example a strip of cotton wool, as such, and to store it, and then supply it to the production process for the tampon. In this case, the absorbent material provided with the functional material can also be bought separately.

If the covering material is to be provided with the functional material, this can likewise take place at the discharge point of a carding machine or in the course of a laying process and/or upon passing into the tampon machine. In this case too, the provision with the functional material can take place in a separate process step. It is also conceivable to store covering materials already provided with the functional material separately and to supply them separately to the production process for the tampon. In this case too, the covering material provided with the functional material can be bought separately.

The provision of the covering material and/or of the absorbent material with the functional material takes place with the aid of an adhesive application head. The application devices may be made to be rigid, mobile, continuous and/or intermittent. In all of the embodiments of the invention a strip of cotton wool or covering material is preferably used as the absorbent material. Furthermore, in the implementation of methods according to the invention the tampon is preferably produced so that the insertion end of the tampon is provided with a dome tapering towards an axial end of a substantially cylindrical absorbent body, the dome being able to be provided with a depression in its crown region extending towards the opposite axial end of the absorbent body, as described for example in EP 2740448 A1.

In particularly preferred embodiments of the invention the functional material is applied to the material web and/or the covering material linearly, in particular along application lines running perpendicularly to the winding axis. In this case sections of the tampon succeeding one another in the axial and/or radial direction are separated from one another in terms of function by separating layers made of the functional material so that a desired effect upon the function of the tampon can be made to be variable in the axial and/or radial direction.

In addition or alternatively, the functional material can be applied in dots or drops to the material web and/or the covering material. In a preferred embodiment of the invention a fluid block is formed by a functional material on an end region of the absorbent body of the tampon facing away from the insertion end so as to thus ensure desired leakage protection.

Within the framework of the invention is has also proven to be particularly advantageous if the functional material is applied to the material web and/or the covering material in the form of intermittent application line sections. Corresponding application line sections may be used for desired guidance of the flow of body fluid along an outer boundary surface of the tampon. However, they may also form the described shells, loops, waves etc. in the absorbent core of the tampon, and they may be applied in the desired manner to the covering material and/or to the material web made of absorbent material.

Desired distribution of the functional material within the tampon and/or on an outer boundary surface of the tampon may be adjusted particularly easily if the material web and/or the covering material is conveyed along a direction of conveyance running approximately perpendicularly to the winding axis when applying the functional material. In this connection an application device designed to apply the functional material is preferably moved in a direction of movement running transversely, in particular approximately perpendicularly, to the direction of conveyance when applying the functional material. Thus, for example, shell- and/or wave-shaped application lines or application line sections can be produced. By means of corresponding application line sections, shell- and/or wave-shaped structures can be created in the region of individual winding layers and/or of the covering material.

It is also possible, however, to form at least one application line section so that it encloses an acute angle (0°< α < 90°) of preferably more than 5° and less than 20° with a plane running perpendicularly to the winding axis. After producing and pressing the winding, an application line section running thus can form one of the shell-shaped, wave-shaped etc. arrangements, as described, from hydrophobic functional material.

As can be gathered from the above explanation of tampons according to the invention and methods according to the invention for producing corresponding tampons, a device according to the invention for producing tampons according to the invention has a device for applying a covering material to a material web made of absorbent material, a winding device for producing a winding from the absorbent material provided with the covering material, and a pressing device for axially and/or radially pressing the winding, and is essentially characterised by an application device designed to provide the absorbent material and/or the covering material with a functional material before and/or during the winding process. The application has an adhesive application head. Particularly preferably, it has at least one, preferably two, three or more application nozzles. Furthermore, the application device can preferably be moved perpendicularly to the direction of conveyance of the material web made of absorbent material. For this purpose, a motor drive may be provided which is controlled according to the desired form of application.

In order to produce intermittent application line sections, the application device may be provided with a blocking device, for example in the form of a solenoid valve, by means of which the application of the functional material can be interrupted. The blocking device may be controlled according to the desired application pattern. For this purpose, the device according to the invention may have a control device programmed according to the desired form of the application pattern. Alternatively or in addition, the control device may also be used to control the motor drive assigned to the application device.

In the following the invention will be explained with reference to the drawings to which reference is explicitly made with regard to all details essential to the invention and not highlighted in any more detail in the description. The drawings show as follows:
- Fig. 1: a schematic illustration of a conventional device for producing tampons, as de-scribed, for example, in EP 2740448 A1,
- Fig. 2: schematic illustrations of devices according to the invention,
- Fig. 3: schematic illustrations of possible application patterns of a functional material,
- Fig. 4: a schematic illustration of an application pattern that can be achieved with additional measures,
- Fig. 5: a schematic illustration of the change to the fluid distribution in the absorbent material or covering material after application of the additive with the aid of the method according to the invention,
- Fig. 6: another schematic illustration of the fluid distribution with the aid of a tampon according to the invention,
- Fig. 7: another schematic illustration of the fluid distribution with a tampon according to the invention,
- Fig. 8: a schematic illustration of the embedding and penetration of the functional material between two strips of material in a tampon according to the invention,
- Fig. 9: a schematic illustration with intermittent application line sections,
- Fig. 10: a schematic illustration of a tampon which is obtained by winding and pressing a material web according to Fig. 9,
- Fig. 11: a schematic illustration of an application line pattern, and
- Fig. 12: a schematic illustration of a tampon obtained by winding and pressing the material web illustrated in Fig. 11.

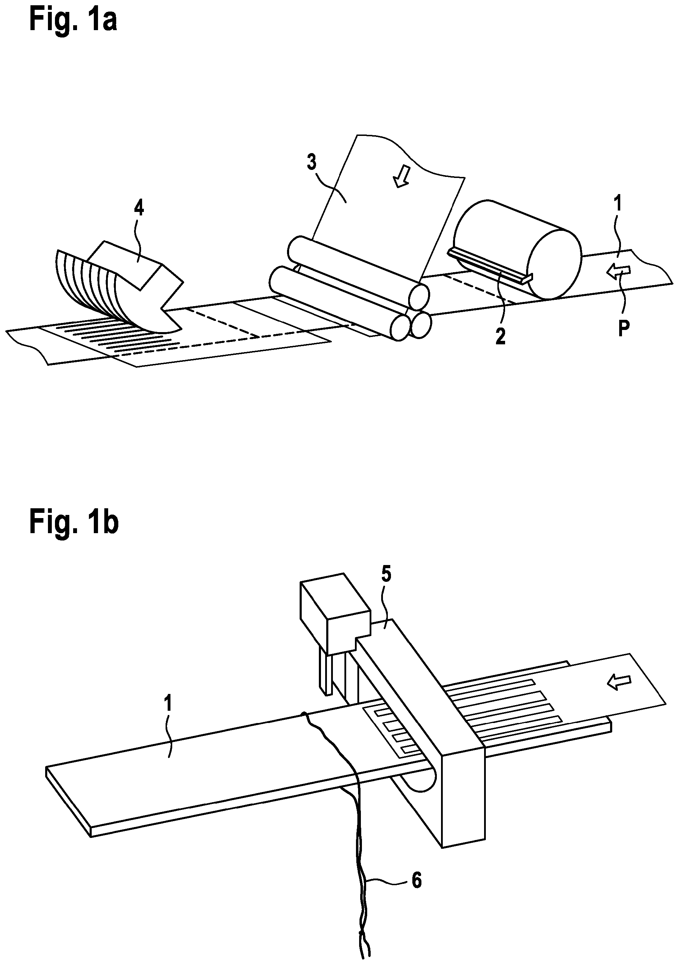

With a device according to Fig. 1a a material web 1 made of absorbent material is drawn into a tampon machine and conveyed as indicated by the arrow P. The material web 1 is pre-cut into material strip sections of the required length with a knife by means of cut lines in a direction running transversely to the direction of conveyance P. The required length is generally 180 to 320 mm, preferably approximately 250 mm. According to the size and the grammage of the tampon to be produced (mini to super plus size), the material web can have a width in a direction running perpendicularly to the direction of conveyance or parallel to the cutting line of 40 to 60 mm, preferably 45 to 50 mm. Advantageously, it has a grammage of 5 g to a maximum of 20 g/m (corresponding approximately to 125 g/m² to a maximum of 335 g/m²). In a preferred embodiment of the invention, the material web made of absorbent material may comprise a fleece made of viscose fibres. However, other absorbent materials or mixtures therefore may also be used, such as for example cotton fibres, cellulose pulp, hydrophilically enhanced synthetic fibres (PP), synthetic foams, natural or synthetic sponges and the like.

The covering material 3 realised, for example, in the form of a fleece, a film, a textile woven and/or knitted fabric is pre-cut into desired lengths (preferably 105 to 145 mm, but at least 90 mm to a maximum of 200 mm) and offset by half the length or more of the material web strip made of absorbent material, as a maximum, however, offset by 4/5 of the length of the section of the material web made of absorbent material subjected to pressure and heat, e.g. by a sealing jaw 4 or by means of ultra-welding to the fleece strip 1.

Hydrophobic as well as hydrophilic materials, such as e.g. plastics (PE, PP, PA, or the like), synthetic substances (viscose, lactate or the like), natural substances (cotton, silk or the like), or mixtures thereof may be used as the base material for the covering material (fleece, film, foams, textile woven/knitted fabric.

The end of the covering material projects over the pre-cut section of the material web made of absorbent material, preferably by approximately 20 to 30 mm, but at least by 10 mm and 60 mm as a maximum. It is thus possible, after producing a winding from the absorbent material provided with the covering material by winding up the section of the material web relative to a winding axis running perpendicularly to the direction of conveyance P, to form the outer boundary surface of the winding from the covering material and for an end section of the covering material to come to rest on a preceding region of the covering material.

The width of the covering material in a direction running perpendicularly to the direction of conveyance and parallel to the direction of cutting may correspond approximately to the width of the material web made of absorbent material. If the width of the covering material is smaller, a tampon may be formed with an insertion end that is not covered by the covering material. If the covering material has a width exceeding the width of the material web made of absorbent material, the insertion end of the tampon can be covered particularly well with the covering material so as to prevent individual fibres from fraying. The width of the covering material is at least 65 and as a maximum 130 % of the width of the material web made of absorbent material.

With the same width the edges of the material web and of the covering material end flush with one another. With a smaller width the edge (thread side) of the material web made of absorbent material facing away from the insertion end ends flush with the edge of the covering material. Therefore, at the insertion end of the material web made of absorbent material a measurement variance forms between the dome-side edge of the material web and the edge of the covering material at which the material web made of absorbent material is not covered by the covering material.

With a larger width the edge of the thread side of the material web made of absorbent material ends flush with the edge of the covering material. Then, a protrusion is formed between the dome-side edge of the material web and the edge of the covering material on the insertion side facing away from the thread side or the dome side of the material web made of absorbent material. With tampons according to the invention the grammage of the covering material is also preferably 10 to 25 g/m², but 50 g/m² as a maximum.

In the next production step, the section of the material web section provided with the covering material passes through a knotting apparatus 5. By means of the knotting apparatus a retrieval cord 6 is looped around the section of the material web. The retrieval cord is knotted with the knotting apparatus (see Fig. 1b).

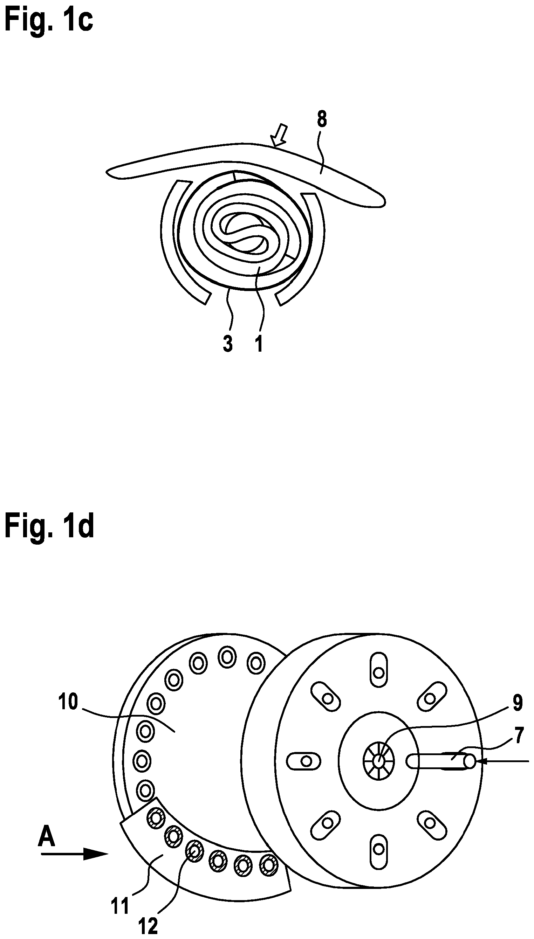

In the next production step, the section of the material web 1 is then wound into a so-called winding 7. In so doing, an overlapping end of the covering material 3 is thermally connected to a layer of the covering material lying underneath with a fusing device 8 (see Fig. 1c).

In the next production step, the winding 7 is passed to an open press 9. After inserting the winding, the press 9 is closed and a blank with grooves of a desired diameter (dependent upon the size, e.g. mini, normal, super, etc., and the type of groove, such as e.g. radial or linear) is pressed (see Fig. 1d).

After slightly opening the press, the blank is pushed backwards out of the press using a pushing-out device and is passed into the tube drum 10. In a head forming unit 11 the blank is pushed by plungers into the head formers 12. Depending on the desired shape of the head (dome shape) and the design of the tampon, 4 to 12 identical or different head formers are incorporated into the head former unit. Finally, the finished tampon is passed to a packaging device where it is wrapped in packaging material and shrink-wrapped therein.

Within the framework of methods according to the invention, the product properties (appearance, feel, absorption capacity, fluid distribution, comfort of wearing, leakage protection, etc.), are positively altered and/or new, additional functions (leakage protection, specific fluid distribution and the like) are generated for the product (tampon) by the thermal application of a preferably adhesive functional material to the material web made of absorbent material and/or to the covering material.

Within the framework of the invention a thermally melting, synthetic or semi-synthetic polymer (bonding agent) can be used as the functional material. The softening point, the processing temperature and the viscosity of the functional material can be varied freely within the framework of the limits already mentioned above. The functional material can be colourless (transparent), white or also coloured (blue, green,...). Advantageously, the functional material that is used is appropriate and approved for a medical application and/or for an application in the hygiene sector.

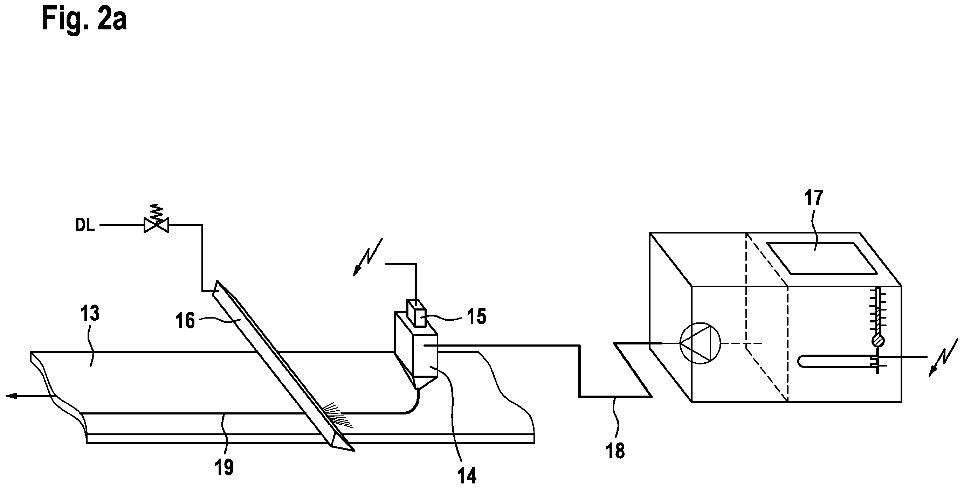

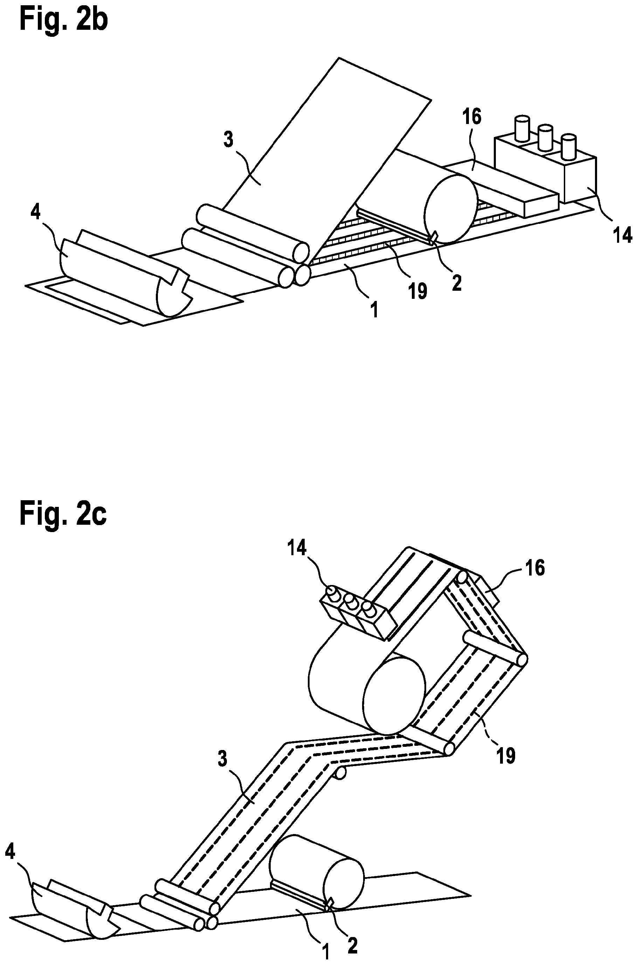

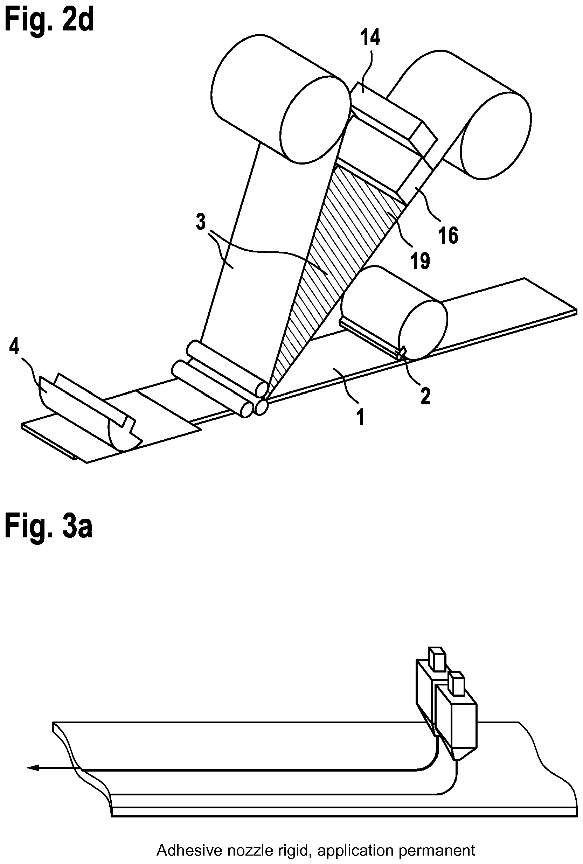

In the following the technique for applying the functional material is explained with reference to Figure 2. In the embodiment of the invention explained with reference to the drawing, the functional material 19 is brought to the desired processing temperature in a heated storage container 17. The conveyance from the storage container to the application device, which is realised in the form of a nozzle in the embodiment explained by the drawing, takes place by means of heated hoses 18. In other embodiments of the invention the functional material may only be heated by the hoses. It may also be conveyed to other application devices such as, for example, stamps, spraying devices or the like.

The application of the functional material to the material web made of absorbent material takes place permanently or intermittently according to the schematic diagram shown in Fig. 2a of devices according to the invention, and for this purpose a solenoid valve controlled by a control device (not shown) according to a predetermined programme is provided. In the embodiment of the invention shown in Fig. 2a, the functional material applied to the material web is cooled by a cooling device 16. The curing time for the functional material can be influenced and varied by the cooling device 16. In the embodiment of the invention shown in the drawing, the cooling device essentially consists of one or more air nozzles arranged in combination and aligned with the material web. By regulating the amount and pressure of the air flow striking the material web, the curing time for the additive is varied.

In the embodiment of the invention shown in Fig. 2b the functional material is applied to the material web using the application device 14 before producing individual sections of the material web, and it is also cooled with the aid of the cooling device 16 before producing the section of the material web.

In the embodiment of the invention shown in Fig. 2c the functional material is applied to the boundary surface of the covering material 3 facing the material web 1 using the application device 14 and is also cooled before bringing together the covering material 3 and the material web 1.

In the embodiment of the invention shown in Fig. 2d the functional material is applied to a boundary surface of a strip of covering material facing the other strip of covering material with the aid of an application device 14 disposed between two strips of a covering material and is cooled with the aid of a cooling device 16.

The application device can have two, three or more application nozzles. The individual nozzles can either be aligned rigidly with just one position of the width of the material web, but they can also be moved transversely to and fro over the width of the material web, in particular perpendicularly to the direction of conveyance of the material web according to a pattern of movement predetermined by a control device. In this way different types of application pattern can be produced.

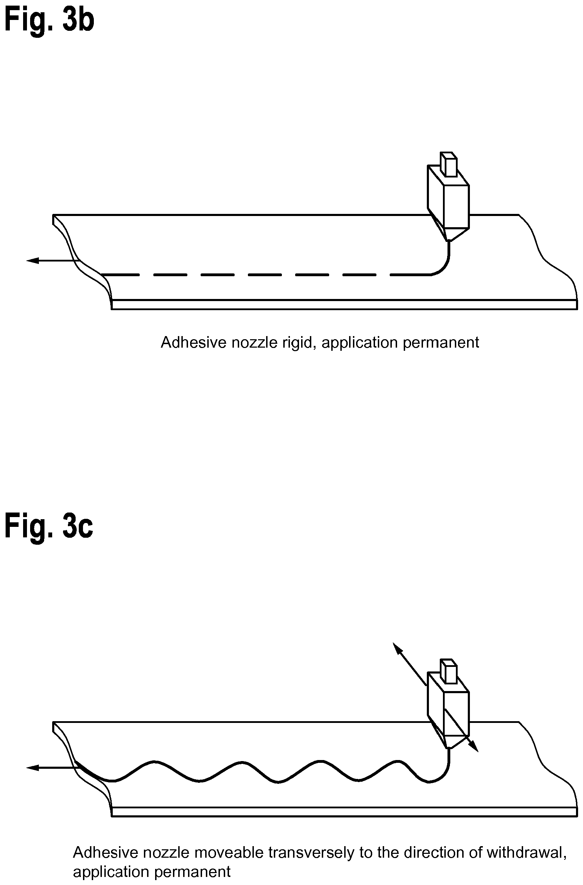

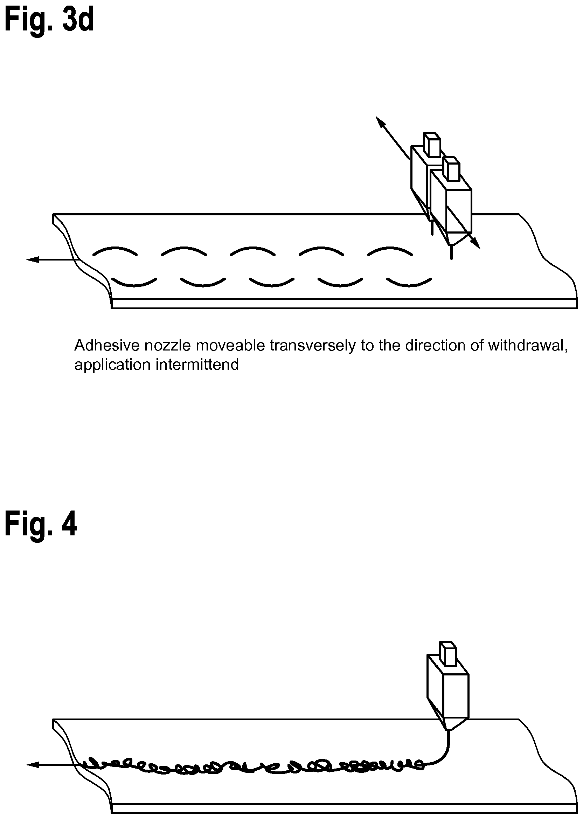

In Fig. 3a an application pattern that can be produced with two application nozzles is shown, with which two lines running parallel to one another and parallel to the direction of conveyance of the material web can be produced. According to Fig. 3b the functional material is applied in the form of intermittent application line sections by appropriate control of the control device realised as a solenoid valve. According to Fig. 3c the functional material is applied in waves by moving the application device in a direction running perpendicularly to the direction of conveyance P. According to Fig. 3d shell-shaped application line sections are produced. In this embodiment of the invention two application nozzles can be moved independently of one another in a direction running perpendicularly to the direction of conveyance.

In the embodiment of the invention shown in Fig. 4 the application pattern of the functional material is varied by additionally using compressed air in the region of the application device.

The core of the invention is to change the product properties by using a functional material. Different product properties can be produced with the aid of the functional material by varying the application parameters. With the predetermined conveyance speed of the material web or the strip of covering material the following application parameters can be changed:
a) amount of functional material applied
b) application temperature for the functional material
c) viscosity of the functional material, dependent upon the type as well as upon the application temperature of the functional material
d) distance between the application nozzles and the material web
e) curing time for the functional material, dependent upon the type of functional material as well as upon the setting of the downstream cooling device.

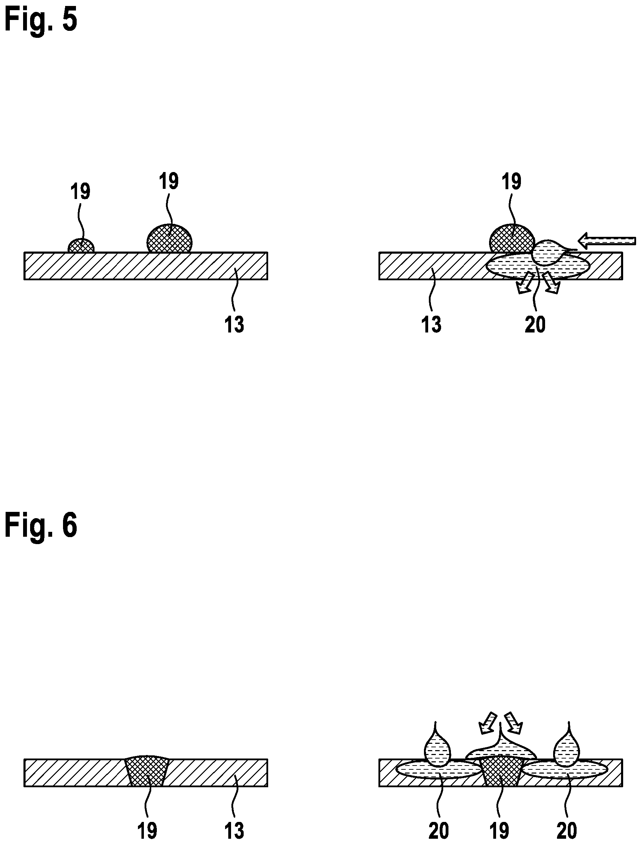

By appropriately setting the application amount, the application temperature, the viscosity, the distance between the application nozzles and the material web, the acceleration of the curing, penetration of the functional material 19 into a hydrophilic material web can be avoided, and a visible, perceptible and functional effect upon the surface of the material web 13 can be achieved. The functional effect can be seen in a guided discharge of a fluid 20 over or through the surface, as shown in Fig. 5.

By specifically setting the application amount, the application temperature, the viscosity and the distance between the nozzles and the material web, as well as by means of a slower curing time obtained by appropriately controlling the cooling device, a high degree of penetration of the functional material 19 into a hydrophilic material web can be achieved. For this purpose, a high application temperature and a low viscosity are set. By correspondingly setting the penetration depth of the functional material the hydrophilic property of the material web can be converted into a hydrophobic property at the application points. In this way the absorption of fluid 20 into the material can be specifically influenced, as shown in Fig. 6. In addition, depending on the application amount and the type properties of the functional material (e.g. cold strength) the material properties, such as for example strength, rigidity, smoothness and appearance can be changed specifically at the application points.

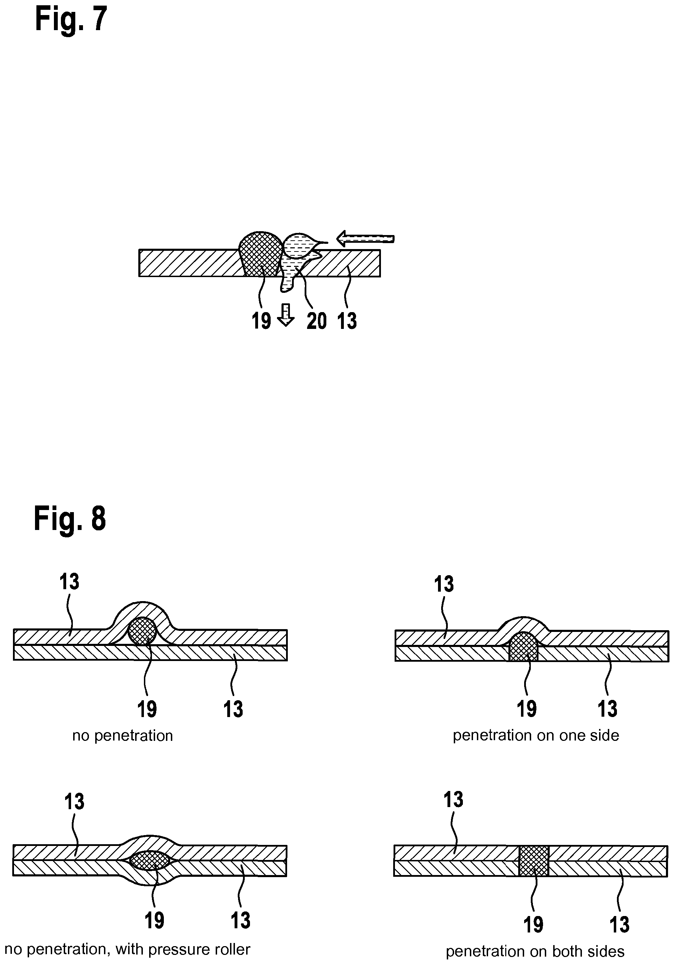

By combining the measures explained by Figs. 5 und 6 and by choosing average settings, it is also possible to vary the effects and material properties, as shown in Fig. 7.

By the temporally controlled bringing together of two material webs 13 (material web made of absorbent material with covering material or also two identical or different strips of covering material) the functional material 19 can be applied and embedded in different ways between these material webs according to the application properties explained by means of Figs. 5 to 7. By using one or more pressure rollers after directly bringing together the material webs, the effect of the functional material upon the material webs or upon the finished product can be seen, additionally influenced and controlled, as illustrated by Fig. 8.

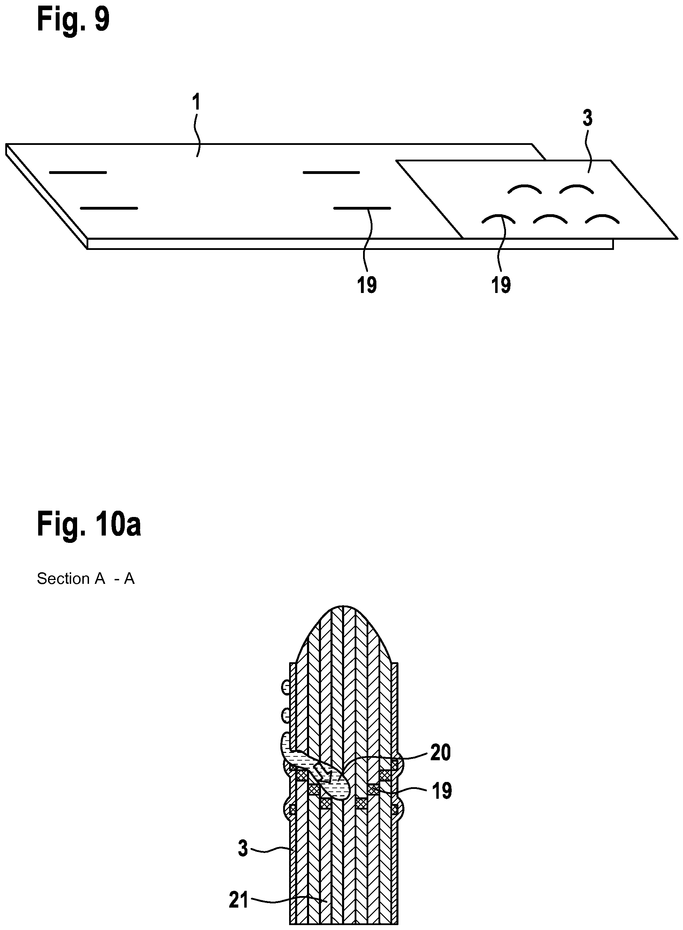

As explained above, the functional material 19 can also be applied to the material web in the form of sections or segments according to the desired structure of the functional material in the finished tampon, as shown in Fig. 9.

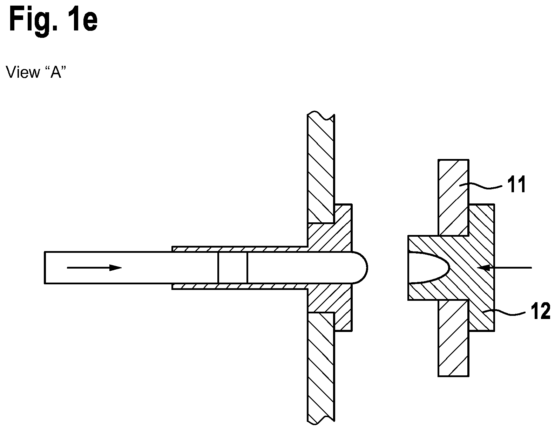

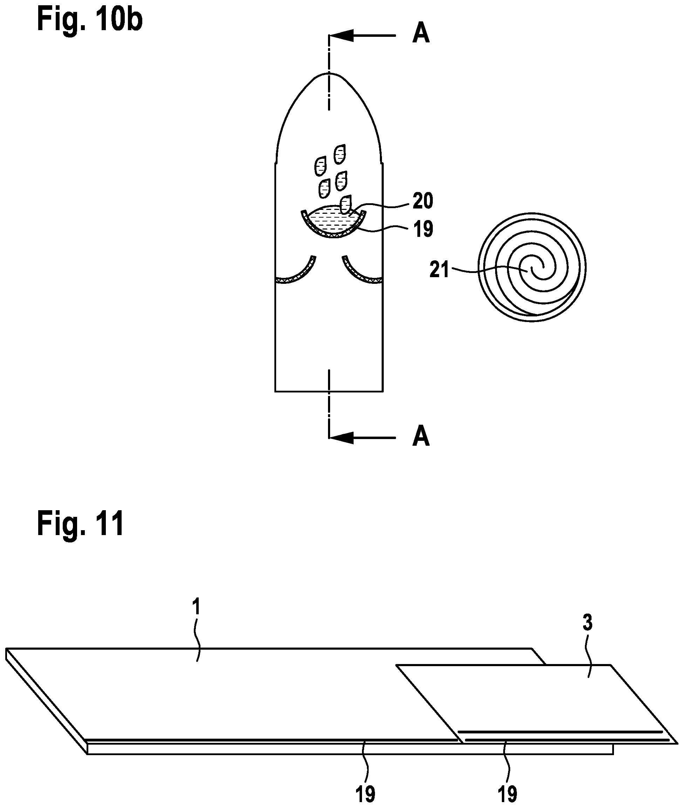

By shaping the section of the material web provided with the covering material to form a winding, and by pressing the winding to form a tampon, as explained by Fig. 1, a collecting or distributing structure which specifically conducts the fluid into the inner hydrophilic layers of the tampon is produced on the surface of the tampon or in the core of the tampon 21 by the overlaying of the individual layers with the functional material application (hydrophobic barriers), as shown in Fig. 10. Here, Fig. 10a shows a sectional illustration of a tampon according to the invention taken along sectional plane A according to Fig. 10b. A fluid 20 that is flowing along is halted by the elevated hydrophobic barriers of the functional material application 19 on the covering material 3 and is specifically discharged into the inner absorbent (hydrophilic) layers in the core of the tampon. By means of further specifically positioned hydrophobic barriers comprised of the functional material application in the core of the tampon 21 (application on the material web made of absorbent material) the fluid is reliably conducted into the interior (into the middle) of the tampon core.

Since in the embodiment of a tampon according to the invention explained by Figs. 9 and 10 the inner absorbent layers are used more specifically and more quickly, the fluid absorption behaviour of the tampon is substantially improved and premature leakage without complete utilisation of the absorptive capacity of the tampon core is prevented.

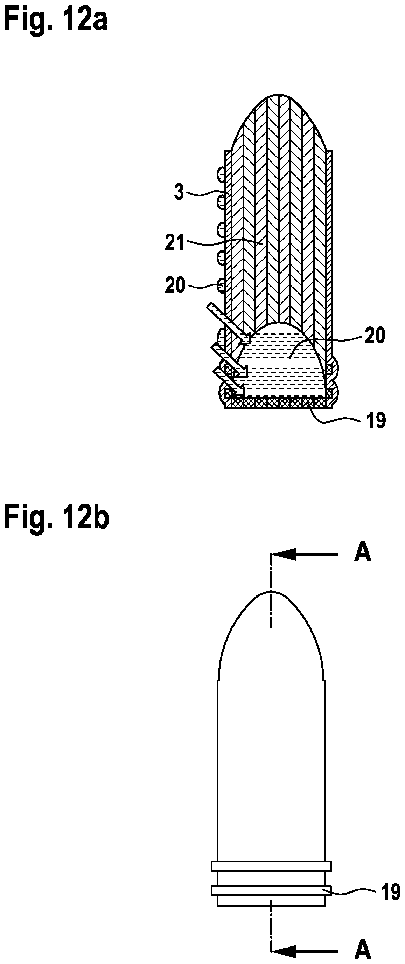

With the aid of the functional material applied according to Figs. 11 and 12, the leakage protection of a tampon can be improved. The functional material is applied to the section of the fleece web 1 and to the covering material 3 in the form of lines running perpendicularly to the winding axis and parallel to the direction of conveyance of the material web 1 according to the desired leakage protection on the side of the material web 1 facing away from the insertion end. The functional material can be applied to the material web 1 as well as to the covering material 3. By shaping the section of the material web to form a winding and by pressing the winding into a tampon according to Fig. 1 a collecting or blocking structure, which specifically holds the fluid in the inner hydrophilic layers of the tampon and does not allow them to pass through at the end of the tampon, is produced on the tampon surface or on the core of the tampon 21 by overlaying the individual layers with the functional material application (hydrophobic barriers).

A fluid 20 which flows along on the surface of the tampon is halted by the elevated hydrophobic barriers of the functional material application 19 on the covering material 3 and is specifically conveyed away into the inner absorbent (hydrophilic) layers (into the core of the tampon) of the tampon. By means of further specifically positioned hydrophobic barriers comprised of an application of additive in the tampon core 21, fluid is prevented from running out at the end of the tampon until the absorptive capacity of the core of the tampon is completely utilised.

The invention is not restricted to the exemplary embodiments explained by means of the drawings. In fact, the functional material can also be applied in dots or drops to the material web and/or to the covering material. Although the application of functional material with adhesive properties has been explained by the drawings, functional material with swelling properties or colour change properties can also be applied.

## Claims

1. A method for producing a tampon comprising a pressed, absorbent material wound up to form a winding and a covering material at least partially covering the outer boundary surface of the winding, wherein a functional material that affects the feel, the look and/or the function of the tampon is provided in the winding and/or the covering material and/or between the winding and the covering material and/or on a surface of the covering material facing away from the winding, wherein a covering material is applied to a material web made of absorbent material, the absorbent material that is thus provided is wound up relative to a winding axis to form a winding such that the covering material at least partially covers the outer boundary surface of the winding, the winding is pressed in the axial and/or radial direction, wherein the absorbent material and/or the covering material is provided with the functional material before and/or during the winding process, **characterized in that** the functional material has a softening temperature as determined according to ASTME28 in glycerine in the range of between 80° C and 200° C and a processing temperature in the range of between 80° C and 280° C and is brought to the desired processing temperature and applied with an adhesive application head.

2. The method according to Claim 1, **characterised in that** the functional material is applied to the material web and/or the covering material linearly, in particular along application lines running perpendicularly to the winding axis.

3. The method according to Claim 1 or 2, **characterised in that** the functional material is applied in dots or drops to the material web and/or the covering material.

4. The method according to Claim 12 or 3, **characterised in that** the functional material is applied to the material web and/or the covering material in the form of intermittent application line sections.

5. The method according to any of Claims 2 to 4, **characterised in that** the material web and/or the covering material is conveyed along a direction of conveyance running approximately perpendicularly to the winding axis when applying the functional material and/or an application device designed to apply the functional material is moved in a direction of movement running transversely, in particular approximately perpendicularly to the direction of conveyance when applying the functional material.

6. The method according to any of Claims 2 to 5, **characterised in that** at least one application line section encloses an acute angle (0°< α < 90°) of preferably more than 5° and less than 20° with a plane running perpendicularly to the winding axis.

## Patentansprüche

1. Verfahren zur Herstellung eines Tampons, umfassend ein gepresstes, saugfähiges Material, das zu einem Wickel aufgewickelt ist, und ein die äußere Begrenzungsfläche des Wickels zumindest teilweise bedeckendes Abdeckmaterial, wobei ein die Haptik, die Optik und/oder die Funktion des Tampons in der Wicklung und/oder dem Abdeckmaterial und/oder zwischen der Wicklung und dem Abdeckmaterial und/oder auf einer der Wicklung abgewandten Oberfläche des Abdeckmaterials vorgesehen ist, wobei ein Abdeckmaterial auf einer Materialbahn aus saugfähigem Material aufgebracht ist, das so bereitgestellte saugfähige Material wird relativ zu einer Wickelachse zu einem Wickel derart aufgewickelt, dass das Abdeckmaterial die äußere Begrenzungsfläche des Wickels zumindest teilweise bedeckt, der Wickel wird in axialer Richtung und/oder radialer Richtung gepresst, wobei das absorbierende Material und/oder das Abdeckmaterial vor und/oder während des Wickelvorgangs mit dem Funktionsmaterial versehen wird, **dadurch gekennzeichnet, dass** das Funktionsmaterial eine Erweichungstemperatur hat bestimmt nach ASTME28 in Glycerin im Bereich zwischen 80° C und 200° C und eine Verarbeitungstemperatur im Bereich zwischen 80° C und 280° C und auf die gewünschte Verarbeitungstemperatur gebracht und mit einem Klebstoffauftragskopf aufgetragen wird.

2. Verfahren nach Anspruch 1, **dadurch gekennzeichnet, dass** das Funktionsmaterial linienförmig, insbesondere entlang senkrecht zur Wickelachse verlaufenden Auftragslinien, auf die Materialbahn und/oder das Abdeckmaterial aufgebracht wird.

3. Verfahren nach Anspruch 1 oder 2, **dadurch gekennzeichnet, dass** das Funktionsmaterial punkt-oder tropfenweise auf die Materialbahn und/oder das Abdeckmaterial aufgebracht wird.

4. Verfahren nach Anspruch 2 oder 3, **dadurch gekennzeichnet, dass** das Funktionsmaterial in Form von intermittierenden Auftragslinienabschnitten auf die Materialbahn und/oder das Abdeckmaterial aufgebracht wird.

5. Verfahren nach einem der Ansprüche 2 bis 4, **dadurch gekennzeichnet, dass** die Materialbahn und/oder das Abdeckmaterial beim Auftragen des Funktionsmaterials entlang einer etwa senkrecht zur Wickelachse verlaufenden Förderrichtung gefördert wird und/oder eine Auftragvorrichtung, die dazu ausgelegt ist, das Funktionsmaterials aufzubringen, beim Aufbringen des Funktionsmaterials in einer quer, insbesondere etwa senkrecht zur Förderrichtung verlaufenden Bewegungsrichtung bewegt wird.

6. Verfahren nach einem der Ansprüche 2 bis 5, **dadurch gekennzeichnet, dass** mindestens ein Auftragslinienabschnitt mit einer verlaufenden Ebene einen spitzen Winkel (0° < a < 90°) von vorzugsweise mehr als 5° und weniger als 20° senkrecht zur Wickelachse einschließt.

## Revendications

1. Procédé de production d'un tampon comprenant un matériau absorbant pressé enroulé pour former un enroulement et un matériau de revêtement recouvrant au moins partiellement la surface limite extérieure de l'enroulement, dans lequel un matériau fonctionnel qui affecte la sensation, l'aspect et/ou la fonction du tampon est assurée dans l'enroulement et/ou le matériau de revêtement et/ou entre l'enroulement et le matériau de revêtement et/ou sur une surface du matériau de revêtement opposée à l'enroulement, un matériau de revêtement étant appliqué sur un nappe de matériau en matériau absorbant, le matériau absorbant ainsi fourni est enroulé par rapport à un axe d'enroulement pour former un enroulement de telle sorte que le matériau de revêtement recouvre au moins partiellement la surface limite extérieure de l'enroulement, l'enroulement est pressé dans l'axe et/ou direction radiale, dans laquelle le matériau absorbant et/ou le matériau de revêtement est pourvu du matériau fonctionnel avant et/ou pendant le processus d'enroulement, **caractérisé en ce que** la fonction le matériau a une température de ramollissement telle que déterminée selon la norme ASTME28 dans la glycérine comprise entre 80°C et 200°C et une température de traitement comprise entre 80°C et 280°C et est amené à la température de traitement souhaitée et appliqué avec une tête d'application d'adhésif.

2. Procédé selon la revendication 1, **caractérisé en ce que** le matériau fonctionnel est appliqué sur la bande de matériau et/ou le matériau de revêtement de manière linéaire, notamment selon des lignes d'application s'étendant perpendiculairement à l'axe d'enroulement.

3. Procédé selon la revendication 1 ou 2, **caractérisé en ce que** le matériau fonctionnel est appliqué par points ou gouttes sur la nappe de matériau et/ou le matériau de revêtement.

4. Procédé selon la revendication 2 ou 3, **caractérisé en ce que** le matériau fonctionnel est appliqué sur la nappe de matériau et/ou le matériau de revêtement sous forme de tronçons de ligne d'application intermittents.

5. Procédé selon l'une quelconque des revendications 2 à 4, **caractérisé en ce que** la bande de matériau et/ou le matériau de revêtement sont convoyés selon une direction de convoyage s'étendant sensiblement perpendiculairement à l'axe d'enroulement lors de l'application du matériau fonctionnel et/ou d'un dispositif d'application destiné à appliquer le matériau fonctionnel est déplacé dans un sens de déplacement s'étendant transversalement, notamment sensiblement perpendiculairement à la direction de transport lors de l'application du matériau fonctionnel.

6. Procédé selon l'une quelconque des revendications 2 à 5, **caractérisé en ce qu'**au moins une section de ligne d'application forme un angle aigu (0°< a < 90°) de préférence supérieur à 5° et inférieur à 20° avec un plan courant perpendiculairement à l'axe d'enroulement.
